# Europäisches Patentamt

(19) **European Patent Office**

**Office européen des brevets**

(11) Publication number: **0 212 641 B1**

## EUROPEAN PATENT SPECIFICATION

(45) Date of publication of patent specification: **10.04.91**

(51) Int. Cl.⁵: **A61K 9/22**

(21) Application number: **86111636.6**

(22) Date of filing: **22.08.86**

(54) Taste masking compositions.

(30) Priority: **26.08.85 US 768981**

(43) Date of publication of application:
**04.03.87 Bulletin 87/10**

(45) Publication of the grant of the patent:
**10.04.91 Bulletin 91/15**

(84) Designated Contracting States:
**AT BE CH DE FR GB IT LI NL SE**

(56) References cited:
**FR-A- 2 118 999**
**US-A- 2 993 837**
**US-A- 3 594 470**
**US-E- 28 316**

**Pharmaceutical Ind., vol. 36, no. 6, June
1974, pages 432-437; B.H. Lippold et al.:
"Freisetzung von Carbutamid aus Arzneifor-
men mit Polymethacrylsäurederivaten"**

(73) Proprietor: **THE PROCTER & GAMBLE COM-
PANY**
**One Procter & Gamble Plaza**
**Cincinnati Ohio 45202(US)**

(72) Inventor: **Damani, Nalinkant Chunilal**
**11510 Reed Hartman Highway**
**Cincinnati Ohio 45241(US)**
Inventor: **Tsau, Josef Hengko**
**5348 Brummel Street**
**Skokie, II. 60076(US)**

(74) Representative: **Wolff, Hans Joachim, Dr.jur.
Dipl.-Chem. et al**
**Beil, Wolff & Beil Rechtsanwälte Postfach 80
01 40 Adelonstrasse 58**
**W-6230 Frankfurt am Main 80(DE)**

## Description

The present invention relates to a drug-polymer composition effective in masking the taste of a bitter drug. The invention further relates to methods for preparing such a composition.

## BACKGROUND OF THE INVENTION

Chewable and lozenge dosage forms are preferred by individuals who have difficulty swallowing tablets and capsules. Frequently, sweetners and flavors, along with diluting agents have been utilized as fillers in an attempt to minimize unpleasant or bitter taste of pharmaceutical active ingredient.

Many drugs, in particular drugs containing amine or amido groups or salts thereof, often have a strong bitter taste. Without proper taste-masking, such drugs cannot be adapted into acceptable tasting chewables and lozenges.

FR-A 2 118 999 discloses entrapment of fine particles of several active ingredients such as penicilline-derivatives by using polymers and copolymers of methacrylat ester derivatives, cellulose acetate or hydroxycellulose in order to control the release of the drug.

US-A 28 316 relates to entrapment compositions of active ingredients having a basic or acidic group being contacted with an aqueous latex of a polymer such polyvinyl and acrylic acids and esters.

Such compositions may have sustained, enteric delayed or controlled release properties.

One example of a bitter amino drug is dimenhydrinate. In addition to its bitter taste, dimenhydrinate produces a numbing effect that is equally as unpleasant.

Taste-masking techniques using various sweeteners, amino acids, acids, flavors and adsorbents have been unsuccessful in masking the taste of dimenhydrinate or if the taste is somewhat masked, the resulting product is therapeutically ineffective. One method involves the enteric coating of bitter tasting drugs with various copolymers. However, enteric coating has been ineffective. The coating of fine particle materials is usually unsuccessful and the coatings of granular particles are readily ruptured by chewing and compression. In addition, most coatings do not have an acceptable in vivo drug releasing mechanism.

Cation-exchange resins have been used to adsorb amine drugs for sustained release action and taste-masking, e.g. as described in the US-A 3 594 470. The widely used cation-exchange resins are polysulfonic acid and polycarboxylic acid polymers. In addition rice endosperm has been used to mask the taste of drugs utilizing its properties of stickiness and insolubility to physically entrap the drug molecules. However, neither technique has been effective in masking the bitter taste or numbing effect associated with dimenhydrinate.

## SUMMARY OF THE INVENTION

The present invention relates to a porous drug-polymer matrix comprising an active ingredient which is dimenhydrinate or salt thereof and a pharmaceutically acceptable copolymer which is a copolymer of methylmethacrylate acid or esters wherein the matrix dissociates in a media having a pH less than 4, thereby releasing the active ingredient into the media. The porous drug-polymer matrix of the present invention is formed by admixing the active ingredient and the pharmaceutically acceptable copolymer, or mixture of copolymers, in the presence of a solvent and then removing the solvent from the mixture to yield a porous matrix. The porous drug-polymer matrix of the present invention is effective in masking the taste of an active ingredient and releasing the active ingredient in the stomach. The present invention further relates to a method for masking the taste of an active ingredient and to a method for preparing such compositions.

## DETAILED DESCRIPTION OF THE INVENTION

In accordance with the present invention, the taste of an active ingredient is masked by admixing the active ingredient and the pharmaceutically acceptable copolymer in the presence of a solvent and then removing the solvent to yield a porous drug-polymer matrix. The porous matrix thus formed is readily distinguished from a product comprising an active ingredient enterically coated with a copolymer by the fact that at a pH of less than 4 the drug polymer matrix releases the active ingredient wherein an enterically coated active ingredient will only be significantly released in an alkaline media.

The copolymer effective in the composition and methods of the present invention is a pharmaceutically acceptable anionic copolymer capable of interacting with an active ingredient to form an a porous drug-polymer maxtrix. The copolymer utilized in the present invention has a plurality of carboxylic acid and ester groups. Such groups are largely responsible for physical or chemical interactions with an active ingredient for effective taste masking properties. The specific copolymer is readily ascertained by one of ordinary skill in the art. Generally the copolymer that is utilized to mask the taste of an active ingredient in accordance with the present invention is pharmaceutically acceptable in terms of safety and toxicity. It is preferred that said copolymer be soluble in a solvent or a mixture of solvents. Such copolymers include methyl methacrylic acids, esters and co-polymers. Such compounds include commercially available materials sold under trade names such as Eudragit S (trademark of Rohm Pharma).

The active ingredient capable of forming a drug-polymer matrix with a copolymer has an amine group capable of physically or chemically interacting with the carboxylic acid and esters thereof groups of the copolymer. The methods for preparing the porous drug-polymer matrix of the present ensures that physical effect, such as molecular inclusion, adsorption and granulation are present to significantly reduce the rate of release of the active ingredient and thereby effectively reduce the bitter taste of the active ingredient in the mouth. Illustrative of such physical or chemical interactions include one or more of the following: partial molecular inclusion, hydrogen bonding, salt formation, ion-pair and complex formations, hydrogen-bonding and granulation. The particular type of interaction depends upon the specific active ingredient of copolymer utilized.

The quantity of active ingredient in the drug-polymer matrix of the present invention is in a dose effective for the treatment intended. Therapeutically effective doses of the active ingredient required to prevent or arrest the progress of the medical condition to be treated, are readily ascertained by one of ordinary skill in the art.

As previously noted, the composition of the present invention may be prepared by mixing the active ingredient with the anionic copolymer or mixture of anionic copolymers in the presence of an appropriate solvent and then removing the solvent. Although not required, a plasticizer such as glycols, sorbitol, vegetable oil may be optionally added. The solvent or a mixture of solvents is added to thoroughly wet the mixture. Preferred solvents include pharmaceutically acceptable solvents wherein both the active ingredient and the copolymer are soluble or swellable. Pharmaceutically preferred solvents include ketones such as acetone, alcohols such as ethanol, esters such as ethyl acetate and their mixtures with or without water. Alternatively, a solution of either the active ingredient or copolymer is prepared in a solvent or a mixture of solvents and the solution is then combined with the remaining components. The solvent is then removed by conventional methods. Such methods include vacuum evaporation, tray drying, spray drying, drum or belt film drying. Elevated temperatures may be employed provided the temperature at which the solvent is removed does not cause the active ingredient or copolymer to decompose. It is preferred that the solvent be removed under vacuum while heating in order to yield a matrix having a porous structure and thereby enhances the release of the active ingredient in acidic media. The porous matrix is preferably ground to the desired granular size and is then utilized in the preparation of tablets, chewing gum, wafers, candy and like.

The ratio of active ingredient to copolymer is dependent upon the degree to which the taste is to be masked. An effective taste masking amount of copolymer is employed. An effective taste masking amount of the copolymer refers to an amount of copolymer sufficient to mask the taste of an active ingredient. Generally a ratio of 1:1 by weight of active ingredient: copolymer is preferred. It is ascertained by one of ordinary skill in the art that the ratio can be optimized by studying the taste and the in vitro release profiles of the active ingredient.

The quantity of solvent employed is not critical and is determined by the method selected for solvent removal. For example, if a film drying process is selected, it may be necessary to use more solvent to make the film prior to solvent removal. However, if vacuum tray drying process is employed, only a minimal amount of solvent is employed.

It is known that artificial sweeteners such as aspartame, cyclamate, saccharin, will reduce the bitter taste of certain drugs. Small amounts of such sweetener(s) and/or a flavoring agent(s) can be incorporated during the process of preparing the porous drug polymer matrix to optimize the taste masking effect.

The copolymer employed in the present invention function as a carrier of the active ingredient, thus preventing the release of the active ingredient only while the matrix is in the mouth, but permitting release of the active ingredient in the stomach, thereby effectively masking the taste of the active ingredient. Thus, in theory and practice, the methods of the present invention are significantly different from a classical enteric coating process. In accordance with enteric coating techniques, an active ingredient or a formulated tablet containing the active ingredient is completely coated by a film of a polymer. The resulting polymeric film is insoluble and non-diffusible to the active ingredient below the intestinal pH of about 1. In order for the enclosed active ingredient to be released, the external coating first must be dissolved or ruptured in an alkaline pH enviroment, namely that of intestine. Thus, an enterically coated active ingredient will not be released into the stomach wherein the pH is less than 5. Further, an enteric coating product must be kept intact and swallowed without breaking the coating. However, the porous drug-polymer matrix of the present invention is generally powdered and may be formulated into chewable tablets, chewy confectionery products and like without any adverse effects on the release of the active ingredient. The taste of the active ingredient in such formulated products, even when masticated during consumption, is not apparent.

The following Example is intended to further illustrate the present invention wherein all parts are parts by weight unless otherwise expressly set forth.

EXAMPLE 1

To a mixture of 15 g. of dimenhydrinate and 15 g. of Eudragit S-100 was added 30 ml. of ethyl alcohol. The mixture was stirred under gentle heat to yield a viscous mass which was thinly spread on a tray and dried at 70° C under vacuum to yield a porous matrix. The matrix was granulated by passing the matrix through a sieve mesh #60. 100 mg. of the granulated matrix was added to a mixture of 0.3 g. sugar, 0.2 g. mannitol, 0.005 g. of magnesium stearate and q.s. color and flavor and the resulting mixture was compressed into a bitterless chewable tablet providing 0.05 g. of dimenhydrinate.

In vitro tests conducted on a selected products produced in accordance with the methods of the present invention demonstrated that release of the active ingredient occurs at a pH of 1.

## Claims

1. A drug-polymer matrix comprising dimenhydrinate as active ingredient and a pharmaceutically acceptable copolymer which is a copolymer of methacrylic acid and its methyl ester wherein the maxtrix dissociates in a media having a pH less than 4, thereby releasing the active ingredient into the media.

2. A drug-polymer matrix according to Claim 1 wherein the pharmaceutically acceptable copolymer is Eudragit S-100.

3. A method for producing a drug-polymer matrix capable of masking the taste of dimenhydrinate and releasing the active ingredient into a media having a pH of less than 4, comprising mixing the active ingredient with a pharmaceutically acceptable copolymer which is a copolymer of methacrylic acid and its methyl ester in the presence of a solvent and removing the solvent to yield the matrix.

4. A method according to Claim 3 wherein the solvent is removed under reduced vacuum and at elevated temperatures.

5. A method according to Claim 4 wherein the solvent is a ketone or an alcohol.

6. A method according to Claims 3 to 5 wherein the copolymer is Eudragit S-100.

Claims for the following Contracting State : AT

1. A method for producing a drug-polymer matrix capable of masking the taste of dimenhydrinate as active ingredient and releasing the active ingredient into a media having a pH of less than 4, comprisig mixing the active ingredient with a pharmaceutically acceptable copolymer which is a copolymer of methacrylic acid and its methyl ester in the presence of a solvent and removing the solvent to yield the matrix.

2. A method according to Claim 1 wherein the solvent is removed under reduced vacuum and at elevated temperatures.

3. A method according to Claim 2 wherein the solvent is a ketone or an alcohol.

4. A method according to any of Claims 1 to 3 wherein the copolymer is Eudragit S-100.

## Revendications

1. Matrice produit pharmaceutique/polymère comprenant du dimenhydrinate comme ingrédient actif et un copolymère pharmaceutiquement acceptable, qui est un copolymère d'acide méthacrylique et de son ester méthylique, la matrice se dissociant dans un milieu ayant un pH inférieur à 4 en libérant ainsi l'ingrédient actif dans le milieu.

2. Matrice produit pharmaceutique/polymère selon la revendication 1, dans laquelle le copolymère pharmaceutiquement acceptable est "Eudragit S-100".

3. Procédé pour produire une matrice produit pharmaceutique/polymère capable de masquer le goût du dimenhydrinate et de libérer l'ingrédient actif dans un milieu ayant un pH inférieur à 4, ce procédé comprenant le mélangeage de l'ingrédient actif avec un copolymère pharmaceutiquement acceptable, qui est un copolymère de l'acide méthacrylique et de son ester méthylique, en présence d'un solvant, et l'enlèvement du solvant pour obtenir la matrice.

4. Procédé selon la revendication 3, dans lequel le solvant est enlevé sous vide réduit et à des températures élevées.

5. Procédé selon la revendication 4, dans lequel le solvant est une cétone ou un alcool.

6. Procédé selon les revendications 3 à 5, dans lequel le copolymère est "Eudragit S-100".

Revendications pour l'Etat contractant suivant : AT

1. Procédé pour produire une matrice produit pharmaceutique/polymère capable de masquer le goût du dimenhydrinate et de libérer l'ingrédient actif dans un milieu ayant un pH inférieur à 4, ce procédé comprenant le mélangeage de l'ingrédient actif avec un copolymère pharmaceutiquement acceptable, qui est un copolymère de l'acide méthacrylique et de son ester méthylique, en présence d'un solvant, et l'enlèvement du solvant pour obtenir la matrice.

2. Procédé selon la revendication 1, dans lequel le solvant est enlevé sous vide réduit et à des températures élevées.

3. Procédé selon la revendication 2, dans lequel le solvant est une cétone ou un alcool.

4. Procédé selon l'une quelconque des revendications 1 à 3, dans lequel le copolymère est "Eudragit S-100".

**Ansprüche**

1. Eine Arzneimittel-Polymermatrix, umfassend Dimenhydrinat als aktiver Wirkstoff und ein pharmazeutisch zulässiges Copolymer, welches ein Copolymer aus Methacrylsäure und deren Methylester ist, worin die Matrix dissoziiert in einem Medium mit einem pH-Wert kleiner als 4, wobei der aktive Wirkstoff in dieses Medium freigesetzt wird.

2. Eine Arzneimittel-Polymermatrix gemäß Anspruch 1, worin das pharmazeutisch zulässige Copolymer Eudragit S-100 ist.

3. Verfahren zur Herstellung einer Arzneimittel-Polymermatrix, die es erlaubt, den Geschmack von Dimenhydrinat abzudecken und den aktiven Wirkstoff in einem Medium mit einem pH-Wert kleiner als 4 freizusetzen, umfassend das Zusammenmischen des aktiven Wirkstoffs mit einem pharmazeutisch zulässigen Copolymer, welches ein Copolymer aus Methacrylsäure und deren Methylester ist, in Gegenwart eines Lösungsmittels und Entfernen desselben, wobei die Matrix erhalten wird.

4. Verfahren gemäß Anspruch 3, worin das Lösungsmittel unter vermindertem Druck und bei erhöhten Temperaturen entfernt wird.

5. Verfahren gemäß Anspruch 4, worin das Lösungsmittel ein Keton oder ein Alkohol ist.

6. Verfahren gemäß den Ansprüchen 3 bis 5, worin das Copolymer Eudragit S-100 ist.

Patentansprüche für folgenden Vertragsstaat : AT

1. Verfahren zur Herstellung einer Arzneimittel-Polymermatrix, die es erlaubt, den Geschmack von Dimenhydrinat als aktiver Wirkstoff abzudecken und den aktiven Wirkstoff in einem Medium mit einem pH-Wert kleiner als 4 freizusetzen, umfassend das Zusammenmischen des aktiven Wirkstoffs mit einem pharmazeutisch zulässigen Copolymer, welches ein Copolymer aus Methacrylsäure und deren Methylester ist, in Gegenwart eines Lösungsmittels und Entfernen desselben, wobei die Matrix erhalten wird.

2. Verfahren gemäß Anspruch 1, worin das Lösungsmittel unter vermindertem Druck und bei erhöhten Temperaturen entfernt wird.

3. Verfahren gemäß Anspruch 2, worin das Lösungsmittel ein Keton oder ein Alkohol ist.

4. Verfahren gemäß einem der Ansprüche 1 bis 3, worin das Copolymer Eudragit S-100 ist.